# EUROPÄISCHE PATENTSCHRIFT

(11) **EP 1 062 032 B1**
(45) Veröffentlichungstag und Bekanntmachung des Hinweises auf die Patenterteilung: **31.03.2004**
(21) Anmeldenummer: 99906124.5
(22) Anmeldetag: 14.01.1999
(51) Int. Cl.: B01J 13/04, A61K 9/16

(54) **VERFAHREN UND VORRICHTUNG ZUM VERKAPSELN VON MIKROBIELLEN, PFLANZLICHEN UND TIERISCHEN ZELLEN BZW. VON BIOLOGISCHEN UND CHEMISCHEN SUBSTANZEN**
METHOD AND DEVICE FOR CAPSULATING MICROBIAL, PLANT AND ANIMAL CELLS OR BIOLOGICAL AND CHEMICAL SUBSTANCES
PROCEDE ET DISPOSITIF POUR ENCAPSIDER DES CELLULES MICROBIENNES, VEGETALES ET ANIMALES OU BIEN DES SUBSTANCES BIOLOGIQUES ET CHIMIQUES

(30) Priorität: 07.03.1998 DE 19809965
(43) Veröffentlichungstag der Anmeldung: 27.12.2000
(73) Patentinhaber: INOTECH AG, 5605 Dottikon (CH)
(72) Erfinder: PLÜSS-WENZINGER, Raphael, CH-7220 Schiers (CH); WIDMER, Fritz, CH-8044 Gockhausen (CH); HEINZEN, Christoph, 8092 Zürich (CH); BRANDENBERGER, Harry, CH-8620 Wetzikon (CH)
(74) Vertreter: Behrmann, Niels, Dipl.-Ing.
(86) Internationale Anmeldenummer: PCT/EP1999/000162
(87) Internationale Veröffentlichungsnummer: WO 1999/044735

(56) Entgegenhaltungen:
- WO-A-96/28247
- DE-A- 2 725 849
- US-A- 4 302 166
- US-A- 4 981 625
- PATENT ABSTRACTS OF JAPAN vol. 018, no. 476 (C-1246), 6. September 1994 & JP 06 154587 A (FREUNT IND CO LTD), 3. Juni 1994

## Beschreibung

Die Erfindung betrifft ein Verfahren zum Verkapseln von mikrobiellen, pflanzlichen und tierischen Zellen bzw. von biologischen und chemischen Substanzen durch eine Düse in kleine, i. w. kugelförmige Teilchen nach dem Oberbegriff des Patentanspruches 1 sowie eine Vorrichtung dafür.

Die Verkapselung von mikrobiellen, pflanzlichen und tierischen Zellen und von biologischen und chemischen Substanzen -- wie Katalysatoren -- ist vor allem in der Biotechnologie und der Medizin zur Immobilisierung von großer Bedeutung. In der Medizin dient die Verkapselung zusätzlich zum Abschirmen vor dem Immunsystem. Durch die Immobilisierung ist es möglich, die Zellen oder den Katalysator im Prozeß zurückzuhalten und gleichzeitig das Produkt zu ernten. Dadurch sind ein verlängerter Nutzen und eine erhöhte Raum-Zeit-Ausbeute möglich. Durch die Abschirmung der Zellen vor dem Immunsystem ist es möglich, einem Patienten körperfremde Zellen zu implantieren, die über längere Zeit einen gewünschten Stoff in den Körper des Patienten abgeben, ohne dass sie durch das Immunsystem des Patienten angegriffen und zerstört werden.

Die Verkapselung von Zellen und Katalysatoren in Biopolymere -- wie Carrageenan oder Alginat -- und synthetische Polymere -- wie Polyacrylamid -- ist eine in Forschungslabors seit einigen Jahren angewandte Methode. In der Literatur werden dafür viele verschiedene Vorrichtungen beschrieben. Eine der effizientesten Methoden ist das Zerteilen eines Strahls durch die Überlagerung einer externen Schwingung auf die Immobilisierungsflüssigkeit. Die Flüssigkeit wird dadurch beim laminaren Ausströmen aus einer Düse in gleichgroße Fraktionen aufgeteilt. Mehrere Methoden zur Schwingungsübertragung werden genutzt oder beschrieben, z.B. Koppelung an einen Vibrator, Piezokristall, Schallwellen.

Nach WO 96 28 247 A wird eine als Vorratsbehälter für das Immobilisierungsgemisch dienende Spritze mit einem Luerlook-Verschluss an einen Pulsationskopf angeschlossen. Zum Transport des Immobilisierungsgemisches wird der Spritzenkolben durch einen mechanischen Vorschub bewegt. Das Immobilisierungsgemisch wird in ein -- durch ein Gestänge mit dem Vibrator verbundenes -- Vibrationsrohr und danach durch eine Düse mit einer Geschwindigkeit gefördert, die nach der Düse zu einem kontinuierlichen Flüssigkeitsstrahl führt. Der aus der Düse austretende Strahl wird durch die Vibration -- einer Frequenz von vorzugsweise 400 bis 900 Hz -so in Schwingung versetzt, dass er in annähernd gleichmäßig große, kugelige Teilchen aufgetrennt wird, die im Abstand von vorzugsweise 100 mm von der Düse in eine gerührte Lösung fallen. Bei diesem kommerziellen Verkapselungsgerät wird die Schwingung durch eine starre Verbindung zu einem Vibrator übertragen. Diese Methode besitzt die Schwierigkeit, dass die Achse des Vibrators und die Achse zur Düse exakt ausgerichtet sein müssen, da sonst Störungen auftreten, welche die Homogenität der Kugelgröße massiv beeinträchtigen. Zudem ist der Vibrator teuer. Auch hat sich durch photographische Analysen und Beobachtungen unter Stroboskoplicht gezeigt, dass bei ordnungsgemäßem Betreiben der Vorrichtung bis etwa 100 mm nach der Düse eine monodisperse und einzelsträngige Kugelkette sichtbar ist. Werden die Kugeln nach etwa 100 mm Fallstrecke im Härtungsbad aufgefangen und danach unter dem Mikroskop untersucht, so wurden sehr oft -- und dies nicht vorhersagbar -- Chargen ohne monodisperse Kugelschar erhalten. In den Proben waren zumeist drei verschiedene Kugelpopulationen in unterschiedlichem Verhältnis; die erste besaß den erwarteten Kugeldurchmesser, die zweite ein doppeltes oder mehrfach größeres Volumen als erwartet, und die dritte hatte eine Form von zwei sich mehr oder weniger stark berührenden Einzelkugeln.

Bei einer Vorrichtung nach DE 27 25 849 A ist der Vorratsbehälter für niedrigschmelzende Substanzen oder aus in Wasser bzw. organischen Lösungsmitteln gelöste, dispergierte oder emulgierte Stoffe über einen flexiblen -von der Metallführung eines elektromagnetischen Vibratorsystems umgebenen -- Schlauch engen Querschnitts mit der Ausflussdüse verbunden; der zwischen ihr und dem Vorratsbehälter durchgängige Schlauch liegt ohne Ausbildung eines Hohlraumes auf der Innenseite der Ausflussdüse dicht an, und die Schlauchwand wird mit Hilfe eines elektromagnetischen Vibratorsystems in Schwingungen versetzt, die sich auf die strömende Flüssigkeit übertragen und die Erzeugung uniformer Tropfen wird durch den Anpressdruck eines Zylinderkörpers auf die Schlauchwand mittels Stellschrauben gesteuert und mittels Stroboskoplampe visuell kontrolliert.

In der US-A-4 981 625 ist über der Füllöffnung eines -- auf einem Sumpf aus flüssigem Stickstoff eine Dampfschicht enthaltenden -- Auffanggefäßes eine ringförmige Elektrode angeordnet, durch welche aus einer Düse kommende Tropfen eines Strahls aus Monomeren geführt werden.

In Kenntnis dieses Standes der Technik hat sich der Erfinder das Ziel gesetzt, ein Verfahren und eine Vorrichtung der eingangs genannten Art zu optimieren, völlig sterile Bedingungen zu schaffen und auch bei kaum zertropfbaren Gemischen nach Möglichkeit eine monodisperse Kugelschar zu erreichen - dies nicht nur in der Luft, sondern auch im Härtungsbad. Zudem sollen sich die entstehenden Kugeln auf der Fallstrecke nicht berühren.

Zur Lösung dieser Aufgabe führen die Lehren der unabhängigen Patentansprüche; die Unteransprüche geben günstige Weiterbildungen an. Zudem fallen in den Rahmen der Erfindung alle Kombinationen aus zumindest zwei der in der Beschreibung, der Zeichnung und/oder den Ansprüchen offenbarten Merkmale.

Nach dem erfindungsgemäßen Verfahren wird als Immobilisierungsgemisch ein Flüssigkeitsstrahl unter laminaren Fließbedingungen durch die Schwingung im Bereich von 300 bis 4000 Hz in gleichgroße Teile getrennt, wobei die Schwingung auf das Immobilisierungsgemisch innerhalb eines Pulsationsraumes durch eine nicht starre Verbindung übertragen wird; die kugelförmigen Teilchen werden in einem elektrischen Feld zwischen der Düse und einem Gegenelement elektrisch so stark aufgeladen, dass sie sich gegenseitig abstoßen.

In der Nähe der Düse wird ein elektrisches Feld aufgebaut, so dass im Flüssigkeitsstrahl ein elektrischer Ladungsfluss entsteht, wodurch die entstehenden Tropfen eine elektrische Ladung aufweisen. Diese Aufladung muss so hoch sein, dass die Kugeln sich wegen der gleichartigen Ladung gegenseitig abstoßen, und sich die anfänglich einzelsträngig vorhandene Kugelkette in viele Teilketten aufteilt. Dazu werden Spannungen vorzugsweise im Bereich von 200 bis 1600 V benötigt.

Durch den Dispergierungseffekt fallen die Kugeln nicht mehr auf einem eng begrenzten Gebiet auf die Oberfläche des Härtungsbades, sondern sie werden weit zerstreut.

Dadurch ist es nun möglich, routinemäßig eine monodisperse Kugelschar nicht nur in der Luft sondern auch im Härtungsbad zu erzielen. Ebenso kann nun auch bei Immobilisierungsgemischen, die wegen ihrer chemischen und physikalischen Eigenschaften kaum oder nur teilweise zertropft werden konnten, oft eine monodisperse Kugelschar erzielt werden.

Eine für dieses Verfahren vorgesehene Vorrichtung zeichnet sich u.a. dadurch aus, dass ein der Düse vorgeordneter und das Immobilisierungsgemisch aufnehmender Pulsationsraum von einem Dauermagneten überlagert und dieser gegenüber einer elektrischen Spule angeordnet ist. Zudem ist ein der Düse für ein Immobilisierungsgemisch in Abstand und außerhalb der Düsenachse nachgeordnetes metallisches Gegenelement zur Bildung eines elektrischen Feldes zwischen diesem und der Düse an eine Hochspannungsquelle angeschlossen. Dieses Gegenelement ist als Metallring ausgebildet, dessen Ringdurchbruch von der Düsenachse durchsetzt sein sollte. Zwischen Düse und Gegenelement bzw. Metallring findet sich ein elektrisches Feld, bevorzugt mit dem oben erwähnten Spannungsbereich.

Beim Teilen des Immobilisierungsgemisches werden durch Überlagerung einer externen Schwingung in gleich große Fraktionen diese Schwingungen auf das Immobilisierungsgemisch innerhalb des Pulsationsraumes übertragen. Eines der genannten beiden Aggregate ist erfindungsgemäß auf einer Membrane vorgesehen, welche die Pulsationskammer überspannt, wobei das andere Aggregat von dem der Pulsationskammer zugeordneten durch einen Luftspalt getrennt ist.

Bei einer anderen Ausgestaltung der Vorrichtung sind Dauermagnet und elektrische Spule der Düse oder ihrer Aufhängung zugeordnet, so dass diese die Pulsation einzuleiten vermag.

Das Prinzip des Vibrators aus Magnet und mit Wechselstrom durchflossener Spule wird aus dem Vibrator herausgenommen, und ein Teil davon ist direkt der Pulsationskammer zugeordnet. Wenn durch die Spule Wechselstrom gesandt wird, wird diese abwechselnd positiv und negativ magnetisiert. Die Magnetwellen interagieren mit dem darunterliegenden Magneten und versetzen diesen in Schwingungen. Letztere werden fast widerstandslos auf die Immobilisierungsflüssigkeit übertragen.

Nach einem weiteren Merkmal der Erfindung erzeugen die mit Wechselstrom durchflossene Spule und der Dauermagnet Schwingungen im bevorzugten Bereich von 300 bis 4000 Hz.

So wird mit einfachen Mitteln eine Miniaturisierung der Schwingungsübertragung ermöglicht bei sehr geringem Material- und Energieaufwand. Die Kosten des Verfahrens und der Vorrichtung können um ein Mehrfaches gegenüber den bisher bekannten Vibrationsverfahren gesenkt werden. Als weiterer Vorteil ist anzusehen, dass die Ausrichtung von Magnet und Spule nicht auf 0,1 mm genau zentriert sein muss. Auch sind keine Achsen vorhanden, die exakt ausgerichtet werden müssen.

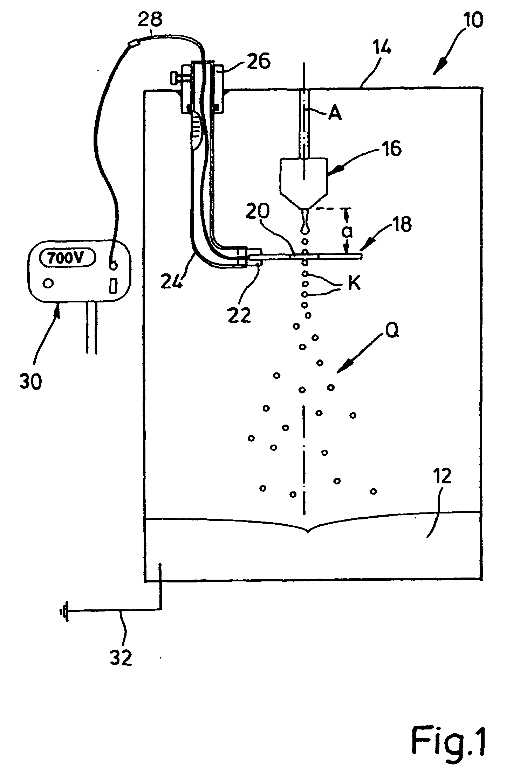

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Seitenansicht einer erfindungsgemäßen Vorrichtung;

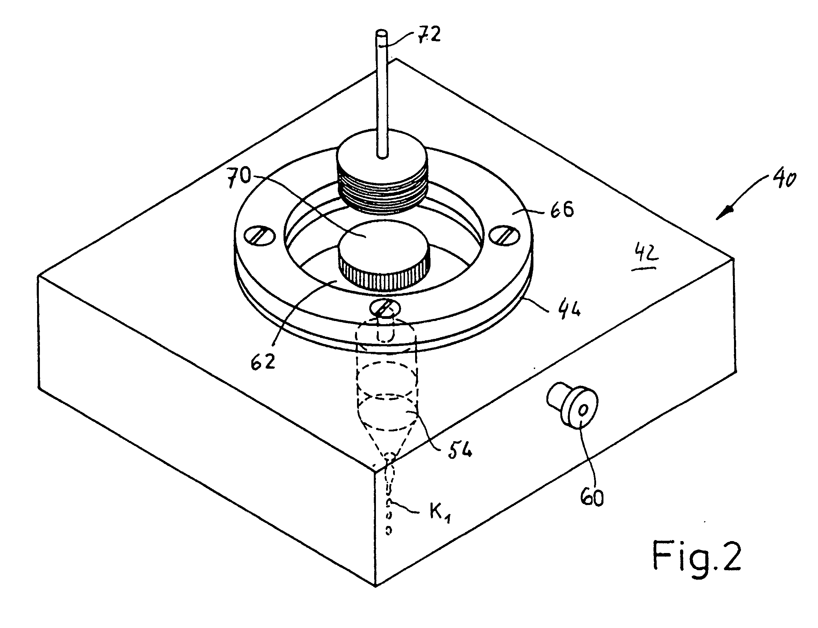

- Fig. 2:: eine Schrägsicht auf eine andere erfindungsgemäße Vorrichtung;
- Fig. 3:: die Draufsicht auf die teilweise geschnittene Vorrichtung der Fig. 2;
- Fig. 4:: den Schnitt durch Fig. 3 nach deren Linie IV-IV.

In einer nur teilweise dargestellten Anlage für das sterile Verkapseln mikrobieller, pflanzlicher und tierischer Zellen ist in einem Reaktor 10 oberhalb eines Härtungsbades 12 sowie unterhalb einer an einem Reaktordeckel 14 hängenden Düse 16 und in Abstand a zu ihr ein Metallring 18 horizontal angebracht, dessen zentrischer Durchbruch 20 von der Düsenachse A durchsetzt ist.

Der Metallring 18 ist mittels eines radialen Halters 22 sowie eines anschließenden Rohres 24 in einem Isolierstutzen 26 im Reaktordeckel 14 befestigt sowie durch eine im Rohr 24 verlegte Leitung 28 an eine Hochspannungsquelle 30 angeschlossen.

Durch die Düse 16 wird ein Verkapselungsgemisch -- bestehend aus einer Immobilisierungsmatrix und Zellen oder Substanzen -- so gefördert, dass ein laminarer Freistrahl entsteht. Durch Überlagern einer Schwingung auf den Freistrahl zerfällt dieser in gleichgroße Tropfen K. Beim Eindringen der Flüssigkeit in ein zwischen dem Metallring 18 und der Düse 16 aufgebautes elektrisches Feld entsteht ein Ladungsfluss in Richtung der Düse 16, so dass die abgetrennten Tropfen K eine elektrische Ladung -- Influenzaufladung -- aufweisen. Durch diese gleichartige Ladung stoßen sich die Tropfen K ab.

Dieser Vorgang führt zu zwei Effekten. Zum einen werden die Tropfen K in axialer Richtung stabilisiert, d.h. sobald sich zwei Tropfen K durch unterschiedliche Fallgeschwindigkeit näher kommen, werden sie durch die Coulomb-Kräfte abgestoßen, und sie können sich nicht berühren. Zum anderen verstärken sich kleinste radiale Versetzungen, und es kommt zu einer Aufweitung der einzelsträngigen Kugelkette zu einem Kegel Q. Durch diesen Effekt wird ein Koagulieren von Tropfen K praktisch verhindert, und im Härtungsbad 12 entstehen vollkommen gleichgroße Partikel. Durch Erdung des Härtungsbades 12 bei 32 werden die Ladungen abgeführt.

In einer Ausgestaltung einer weiteren Anlage für das sterile Verkapseln mikrobieller, pflanzlicher und tierischer Zellen ist oberhalb des Härtungsbades 12 eine beispielsweise rechteckige Tragplatte 40 der Dicke b angeordnet mit in das Zentrum ihrer Oberfläche 42 eingeformter Vertiefung 44 der Tiefe t; letztere entspricht etwa einem Drittel der Plattendicke b.

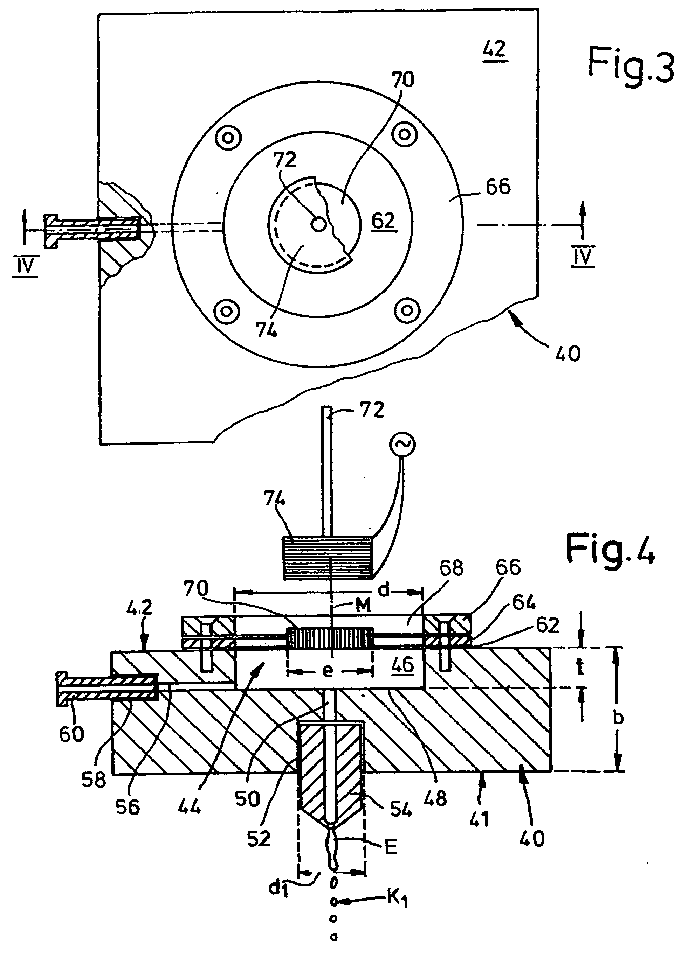

Die Vertiefung 44 ist gemäß Fig. 4 von einer kreisförmigen Umfangswand 46 des Durchmessers d begrenzt, und vom Mittelpunkt ihres Bodens 48 geht eine Bohrung 50 aus. Diese mündet andernends in einer -- in die Unterfläche 41 der Tragplatte 40 eingebrachten -- napfartigen Einformung 52 des Durchmessers d, (etwa ein Drittel d), in welcher eine mit jener Bohrung 50 verbundene Düse 54 sitzt. Zudem führt in der Ebene des Bodens 48 ein Radialkanal 56 zu einem seitlichen Sackloch 58 für einen Anschlussstutzen 60.

Der Vertiefung 44 ist ein auf der Plattenoberfläche 42 -- unter Zwischenschaltung einer Membrane 62 und einer Dichtung 64 -- festgelegter Druckring 66 zugeordnet; dieser ist -- wie auch die Dichtung 64 -- mit einem Innendurchbruch 68 des Durchmessers d ausgestattet und spannt die einen Scheibenmagneten 70 tragende Membrane 62 über der Vertiefung 44 auf. Der Durchmesser e des Scheibenmagneten 70 ist etwas länger als der Durchmesser d₁ der Einformung 52 für die Düse 54.

In Abstand zum Scheibenmagneten 70 hängt -- zu dessen Mittelachse M zentriert -- an einer Halterung 72 eine elektrische Spule 74. Scheibenmagnet 70 und die mit Wechselstrom durchflossene Spule 74 bilden einen Vibrator; wenn durch die Spule 74 Wechselstrom geleitet wird, wird sie abwechselnd positiv und negativ magnetisiert. Die Magnetwellen wirken auf den darunterliegenden Scheibenmagneten 70 und versetzen diesen mitsamt der Membrane 62 in Schwingungen.

In die eine Pulsationskammer bildende Vertiefung 44 ist durch den Radialkanal 56 eine Immobilisierungsflüssigkeit eingeleitet worden, auf welche die Schwingungen fast widerstandslos übertragen werden. Das Einbringen dieses Immobilisierungsgemisches erfolgt mittels eines mechanischen Vorschubes oder durch Luftdruck in die Pulsationskammer oder Vertiefung 44; von dort wird das Immobilisierungsgemisch durch die Düse 54 gedrückt. Der dort entstehende Strahl E zerfällt kurz nach dem Austritt aus der Düse 54 entsprechend der Frequenz der überlagerten Schwingung in gleichgroße Kugeln K₁. Bei etwa 700 Hz entstehen unter optimalen Bedingungen 700 der gleichgroßen Kugeln K₁ je Sekunde, wobei die Homogenität der Kugelgestalt dank der reibungslosen Übertragung ausgezeichnet ist. Messungen haben gezeigt, dass die benötigte Leistung unter 0,2 W liegt.

Bei einer nicht dargestellten Ausführung ist der Dauermagnet 70 oder die Spule 74 unmittelbar an der Düse 54 vorgesehen und das jeweils andere Aggregat unter Bildung eines Luftspaltes zugeordnet.

## Patentansprüche

1. Verfahren zum Verkapseln von mikrobiellen pflanzlichen und tierischen Zellen bzw. von biologischen und chemischen Substanzen durch eine Düse (16, 54) in kleine, i. w. kugelförmige Teilchen (K, K₁) durch Vibration eines Immobilisierungsgemisches, das durch Überlagerung einer externen Schwingung in gleichgroße Fraktionen geteilt wird, wobei die kugelförmigen Teilchen unter sterilen Bedingungen behandelt werden,
**dadurch gekennzeichnet,**
**dass** als Immobilisierungsgemisch ein Flüssigkeitsstrahl unter laminaren Fließbedingungen durch die Schwingung im Bereich von 300 bis 4000 Hz in gleichgroße Fraktionen geteilt wird, wobei die Schwingung auf das Immobilisierungsgemisch innerhalb eines Pulsationsraumes (44) durch eine nicht starre Verbindung übertragen wird, sowie dass die kugelförmigen Teilchen in einem elektrischen Feld zwischen der Düse (16) und einem Gegenelement (18) elektrisch so stark aufgeladen werden, dass sie sich gegenseitig abstoßen.

2. Verfahren nach Anspruch 1, **gekennzeichnet durch** Schwingungen von etwa 700 Hz.

3. Verfahren nach Anspruch 1, **dadurch gekennzeichnet, dass** die kugelförmigen Teilchen unterhalb des Gegenelements (18) und oberhalb eines Härtungsbades (12) unter Bildung eines Kegels auseinandergetrieben werden.

4. Verfahren nach Anspruch 1, **dadurch gekennzeichnet, dass** die elektrische Spannung zwischen der Düse (16) und dem Gegenelement (18) im Bereich von 200 bis 1600 V liegt.

5. Verfahren nach Anspruch 1 oder 3, **dadurch gekennzeichnet, dass** durch Veränderung der Lage und Geometrie des Gegenelements (18) die Abstoßung der kugelförmigen Teilchen (K) variiert wird.

6. Vorrichtung zum Verkapseln von mikrobiellen pflanzlichen und tierischen Zellen bzw. von biologischen und chemischen Substanzen durch eine Düse (16) in kleine, i.w. kugelförmige Teilchen (K), zur Durchführung des Verfahrens nach einem der voraufgehenden Ansprüche, **dadurch gekennzeichnet, dass** ein der Düse (18) vorgeordneter und das Immobilisierungsgemisch aufnehmender Pulsationsraum (44) von einem Dauermagneten (70) überlagert und dieser gegenüber einer mit Wechselstrom durchflossenen Spule (74) angeordnet ist, dass zudem eine der Düse (16) für ein Immobilisierungsgemisch in Abstand (a) und außerhalb der Düsenachse (A) nachgeordnetes metallisches Gegenelement zur Bildung eines elektrischen Feldes zwischen diesem und der Düse an eine Hochspannungsquelle (30) angeschlossen, das Gegenelement als Metallring (18) ausgebildet und dessen Ringdurchbruch (20) von der Düsenachse (A) durchsetzt ist.

7. Vorrichtung nach Anspruch 6, **dadurch gekennzeichnet, dass** der Metallring (18) radial an eine isoliert angebrachte Halterung (22, 24) angeschlossen ist, die einen radialen Halter (22) sowie ein anschließendes Rohr (24) für eine in ihm verlaufende Leitung (28) zwischen Hochspannungsquelle (30) und Metallring (18) enthält.

8. Vorrichtung nach Anspruch 6, **dadurch gekennzeichnet, dass** die Schwingung durch die Spule (74) und den Dauermagneten (70) erzeugbar ist, wobei sich eines der beiden Aggregate auf einer die Pulsationskammer überspannenden Membrane (62) befindet.

9. Vorrichtung nach einem der Ansprüche 6 bis 8, **dadurch gekennzeichnet, dass** die Spule (74) und der Dauermagnet (70) voneinander durch einen Luftspalt getrennt festgelegt sind.

## Claims

1. Method for encapsulating microbial plant and animal cells or biological and chemical substances through a nozzle (16, 54) in small, substantially spherical particles (K, K₁) by vibration of an immobilising mixture which is divided into fractions of equal size by superimposing an external oscillation, wherein the spherical particles are treated under sterile conditions, **characterised in that** as the immobilising mixture a liquid jet is divided into fractions of equal size under laminar flow conditions by the oscillation within the range from 300 to 4000 Hz, wherein the oscillation is transmitted to the immobilising mixture within a pulsation chamber (44) through a non-rigid connection, and **in that** the spherical particles are electrically charged in an electric field between the nozzle (16) and a counter-element (18) so highly that they repel each other.

2. Method according to claim 1, **characterised by** oscillations of about 700 Hz.

3. Method according to claim 1, **characterised in that** the spherical particles are driven apart below the counter-element (18) and above a curing bath (12), forming a cone.

4. Method according to claim 1, **characterised in that** the voltage between the nozzle (16) and the counter-element (18) is within the range from 200 to 1600 V.

5. Method according to claim 1 or 3, **characterised in that** by altering the position and geometry of the counter-element (18) the repulsion of the spherical particles (K) is varied.

6. Device for encapsulating microbial plant and animal cells or biological and chemical substances through a nozzle (16) in small, substantially spherical particles (K), for carrying out the method according to any of the preceding claims, **characterised in that** a pulsation chamber (44) mounted in front of the nozzle (18) and receiving the immobilising mixture is capped by a permanent magnet (70) and the latter is arranged opposite a coil (74) through which alternating current flows, **in that** also a metal counter-element arranged behind the nozzle (16) for an immobilising mixture at a distance (a) and outside the nozzle axis (A) is connected to a high-voltage source (30) for forming an electric field between the counter-element and the nozzle, the counter-element is designed as a metal ring (18) and the nozzle axis (A) passes through its ring aperture (20).

7. Device according to claim 6, **characterised in that** the metal ring (18) is radially connected to a support (22, 24) which is mounted in insulated fashion and which includes a radial holder (22) and an adjoining pipe (24) for a line (28) running in it between high-voltage source (30) and metal ring (18).

8. Device according to claim 6, **characterised in that** the oscillation can be generated by the coil (74) and the permanent magnet (70), wherein one of the two units is located on a diaphragm (62) spanning the pulsation chamber.

9. Device according to any of claims 6 to 8, **characterised in that** the coil (74) and the permanent magnet (70) are fixed and separated from each other by an air gap.

## Revendications

1. Procédé d'encapsulation de cellules microbiennes végétales et animales ou de substances biologiques et chimiques au moyen d'une buse (16, 54) en particules (K, K₁) sensiblement sphériques sous l'effet de la vibration d'un mélange immobilisé qui, sous l'effet de l'interférence d'une vibration externe, est divisé en fractions de taille identique, les particules sphériques étant traitées dans des conditions stériles,
**caractérisé en ce que**
en tant que mélange immobilisé, un jet de liquide, dans des conditions de flux laminaires, est divisé en fractions de taille identique sous l'effet de la vibration dans le domaine de 300 à 4 000 Hz, la vibration étant transmise sur le mélange immobilisé à l'intérieur d'un espace de pulsation (44) par l'intermédiaire d'une liaison non rigide, ainsi qu'en ce que les particules sphériques sont chargées électriquement dans un champ électrique entre la buse (16) et un élément complémentaire (18) avec une intensité telle qu'elles se repoussent mutuellement.

2. Procédé selon la revendication 1, **caractérisé par** des vibrations de 700 Hz environ.

3. Procédé selon la revendication 1, **caractérisé en ce que** les particules sphériques sont repoussées mutuellement en formant un cône en dessous de l'élément complémentaire (18) et au-dessus d'un bain de durcissement (12).

4. Procédé selon la revendication 1, **caractérisé en ce que** la tension électrique entre la buse (16) et l'élément complémentaire (18) se situe dans le domaine de 200 à 1 600 V.

5. Procédé selon la revendication 1 ou 3, **caractérisé en ce que** la variation du positionnement et de la géométrie de l'élément complémentaire (18) permet de faire varier le repoussement des particules sphériques (K).

6. Dispositif d'encapsulation de cellules microbiennes végétales et animales ou de substances biologiques et chimiques au moyen d'une buse (16) en particules (K) sensiblement sphériques, destiné à la mise en oeuvre du procédé selon l'une quelconque des revendications précédentes, **caractérisé en ce qu'**un aimant permanent (70) est superposé à un espace de pulsation (44), disposé en amont de la buse (18) et destiné à recevoir le mélange immobilisé, et ledit aimant est agencé en face d'une bobine (74) traversée par un courant alternatif, **en ce que**, en plus, un élément complémentaire métallique pour un mélange immobilisé, lequel est disposé en aval et à une distance (a) de la buse (16) et en dehors de l'axe de la buse (A), est raccordé à une source de haute tension (30) pour générer un champ électrique entre ledit élément complémentaire et la buse, l'élément complémentaire est conçu sous forme de bague métallique (18) et l'axe de la buse (A) passe à travers le passage circulaire (20) de ladite bague métallique.

7. Dispositif selon la revendication 6, **caractérisé en ce que** la bague métallique (18) est raccordée radialement à un dispositif de support (22, 24), qui est monté de manière isolée et qui contient un support (22) radial, ainsi qu'un tube (24) raccordé, à travers lequel passe une ligne (28) agencée entre la source de haute tension (30) et la bague métallique (18).

8. Dispositif selon la revendication 6, **caractérisé en ce que** la vibration peut être générée par la bobine (74) et l'aimant permanent (70), l'un des deux organes étant disposé sur une membrane tendue au-dessus de la chambre de pulsation.

9. Dispositif selon l'une quelconque des revendications 6 à 8, **caractérisé en ce que** la bobine (74) et l'aimant permanent (70) sont séparés l'un de l'autre par un entrefer.
